# EUROPEAN PATENT APPLICATION

(11) **EP 4 437 987 A1**
(43) Date of publication of application: **02.10.2024**
(21) Application number: 24162277.8
(22) Date of filing: 08.03.2024
(51) Int. Cl.: A61B 18/12, A61B 17/32

(54) **ELECTROSURGICAL GENERATOR**

(30) Priority: 29.03.2023 US 202363455417 P; 21.04.2023 DE 202023110239 U
(71) Applicant: Olympus Winter & Ibe GmbH, 22045 Hamburg (DE)
(72) Inventor: Janich, Fabian, 14469 Potsdam (DE); Dijkstra, Jelle, 12205 Berlin (DE); Ramin, Daniel, 14558 Nuthetal (DE)
(74) Representative: Glawe, Delfs, Moll

(57) **Abstract**

Electrosurgical generator for providing a dual frequency power output in a low and a high frequency range to an output socket (16) for an electrosurgical instrument (19), comprising an inverter unit configured to simultaneously generate said dual frequency power output. The inverter unit is a multilevel inverter (3) comprising a low frequency driving unit (41) configured for forming a low frequency reference signal, a high frequency driving unit (42) configured for forming a HF reference signal, and a modulation unit (6) configured for forming a modulated combined reference signal by modulating the low frequency reference signal onto the high frequency reference signal. The modulated combined reference signal is supplied to the multilevel inverter for generating the dual frequency power output. Thereby, an efficient way for providing highfrequency energy in the low frequency (ultrasound) band as well as the high frequency (radiofrequency) band by means of a single inverter is formed.

## Description

The invention relates to an electrosurgical generator configured to output a high-frequency alternating voltage to at least one electrosurgical instrument. The electrosurgical generator comprises an inverter unit generating a dual frequency output in a low frequency range as well as in a high frequency range to at least one output socket configured for connection of an electrosurgical instrument. The electrosurgical generator shall further be configured to provide ultrasound energy for respective ultrasound-surgical instruments.

An electrosurgical generator supplies energy to an electrosurgical instrument, the type of energy being in particular ultrasound energy and/or high frequency energy. The electrosurgical instrument applies said energy to a tissue to be treated, either directly like in the case of electroscalpel or electrocauter or indirectly by means of a transducer which converts the energy in the ultrasound frequency range to corresponding movements of an actuating element of the instrument. The ultrasound energy is typically provided in the frequency range of about 20 kHz up to 200 kHz and the high frequency energy is higher and typically provided in the radiofrequency range of about 200 kHz to up to 4,000 kHz. Providing said energy results in local heating of the tissue. Thereby, the tissue is cut or severed by heating, and the tissue is removed by thermal resection. A major advantage of electrosurgery is that bleeding can be stopped at the same time as the cutting is made by closing the affected vessels, thereby achieving a coagulation effect.

Different kind of applications require different electrosurgical instruments. Particularly, electrosurgical instruments operating in the ultrasound frequency range (ultrasound instruments) have an advantage in that a minimum of thermal spread is generated, thereby minimizing adverse impact on adjacent tissue. Various kinds of different ultrasound instruments are used, and for specific tasks there also exist electrosurgical instruments that are supplied with both, energy in the ultrasound frequency range as well as the radiofrequency range (e.g. "Thunderbeat" instrument of Olympus Medical Inc.).

Supplying electrosurgical instruments with energy in the ultrasound frequency range and in the radiofrequency range traditionally requires two different electrosurgical generators or providing a "combined" generator having two energy supplying devices (e.g. inverters), one for supplying energy in the ultrasound frequency range and one other for supplying energy in the radiofrequency range. Both energies are supplied to respective outputs for connection of the respective electrosurgical instrument. The outputs may be separate or they may be integrated into a multi-output having connectors for both kinds of energy. Due to the dual energy generation, such a combined electrosurgical generator is rather bulky and expensive, whereas employing of two separate electrosurgical generators requires even more space and creates additional difficulties in terms of synchronizing both generators which is of special concern in case of a hybrid instrument requiring both kinds of energy, like said "Thunderbeat" instrument.

It is thus an object of the invention to provide an electrosurgical generator allowing an improved supply of energy in the low frequency band and in a high-frequency band.

The solution according to the invention resides in an electrosurgical generator according to the features of the independent claim. Advantageous developments are the subject matter of the dependent claims.

In an electrosurgical generator configured to provide a dual frequency power output in a low frequency range as well as in an high frequency range to at least one electrosurgical instrument, comprising a control unit and an inverter unit configured to simultaneously generating said dual frequency power output that is supplied to at least one output socket for connection of at least one electrosurgical instrument, it is provided according to the invention that the inverter unit is configured as a multilevel inverter being driven by an inverter controller, said inverter controller comprising:
- a low frequency driving unit configured for forming a low frequency reference signal for the low frequency output of the electrosurgical generator,
- a high frequency driving unit configured for forming a HF reference signal for the high frequency output of the electrosurgical generator, and
- a modulation unit configured for forming a modulated combined reference signal by modulating the low frequency reference signal onto the high frequency reference signal, wherein the modulated combined reference signal is supplied to the multilevel inverter for generating the dual frequency power output.

The core aspect of the invention is to provide an efficient way for generating and delivering high-frequency energy in the low frequency band as well as the high frequency band by means of a single inverter. This is achieved by forming a combined control signal for the inverter. Owing to the fact that the inverter is configured as a multilevel inverter it can create waves of selectable frequency and waveform, thereby allowing formation of a combined wave which is being formed according to a reference signal created by modulating the low-frequency reference signal on the high-frequency reference signal. The invention has realized that, by using such a combined, modulated reference signal for controlling the multilevel inverter, formation of two different frequencies at once can be achieved by just a single inverter. Thereby space and cost saving are achieved as opposed to conventional concepts requiring two inverters. Further, having both frequencies generated by the same inverter allows for a single connection line within the generator to its output(s). Having a single connection requires less space and facilitates electromagnetic shielding for an effective reduction of EMI (electromagnetic interference).

Further, a multilevel inverter allows for generation of a plurality of voltage levels with a rather fine voltage stepping. Details of the multilevel inverter technology in the field of electrosurgical generators are comprised in applicant's other patent application DE 10 2021 122 282 A1 and DE 10 2021 122 284 A1 which are incorporated herein by reference. Accordingly, by employing such a multilevel inverter a precisely controlled generation of both frequencies and conveying can be achieved which enables a more precise filtering for recovering both frequencies to eventually drive the electrosurgical instrument. Unwanted harmonics can be avoided and/or reduced thereby which improves driving quality of the electrosurgical instrument.

Moreover, by virtue of this kind of generating of two frequencies at once any adverse synchronization issues that may be encountered traditionally by using separate inverters are effectively avoided.

Depending on the location where the recovering of the two original frequencies (frequency splitting) is to be performed, a single connection cable leading to the electrosurgical instrument can be applied provided the device performing the frequency splitting is located at the instrument itself or within the cable near the instrument. This avoids the necessity for providing a second wire connection for the second frequency, thereby easing handling of the electrosurgical instrument.

An additional benefit of the modulation is that the type of connection cable can be specifically tailored of for best HF characteristics in the range of the high frequency onto which the low frequency signal is modulated. As in practical terms the low frequency and the high frequency are separated from each other by one order of magnitude (factor 10) or more, providing a wiring connection tailored to both, the low and high frequency, was difficult and often required compromises. Owing to the invention, the frequency spectrum to be conveyed by the wiring connection is a rather narrow band centered around the high frequency which much facilitates defining highly tailored HF characteristics, thereby further increasing effectiveness of the wiring connection. - This also aids in achieving a better EMI protection.

A further advantage of this narrow band being centered around the high frequency is that transformers, in particular isolation transformer which are required by regulation for electrosurgical generators, are operating more effective at such higher frequencies. This is of particular importance for effectiveness of the low frequency generation and its conveying to the instrument.

In the following, some expressions used within the context of the invention are explained:
The inverter unit is a device to provide the actual high-frequency alternating voltage output for the electrosurgical instrument to be connected to the output socket. Frequency, phase and usually also amplitude and/or shape of the high-frequency voltage generated by the inverter unit is controlled by a reference signal as used by the (inverter) controller.

A multi-level inverter is an inverter unit being enabled to emit an output voltage at various levels, as opposed to providing On/Off output only with positive and/or negative polarity. Typical topologies are, but not limited to, cascaded H-bridge, neutral clamped and flying capacitor.

In the context of the present application the term "high frequency" relates to frequencies in the range of 200 kHz to 4000 kHz which is also being known as radiofrequency range (RF). The term "low frequency" relates to the ultrasound frequency range (US) which is typically in the range of 20 kHz to 200 kHz. The voltage at which these frequencies are output by the electrosurgical generator is typically a high voltage which in the context of the present application is considered as having an amplitude in particular up to 10 kV, preferably up to 4000 Volt and further preferably more than 100 Volt.

Advantageously, a reconstruction unit is provided comprising a frequency splitter having two outputs, a first output connected to a high-frequency supply line and a second output connected to a low -frequency supply line, the supply lines being configured for supplying the output power to the at least one electrosurgical instrument. Thereby, the low-frequency can be split off and an efficient recovering of the original low and high frequencies can be achieved, thereby being enabled to provide the electrosurgical instrument with the respective proper frequency.

Preferably, the frequency splitter comprises programmable filters for the low-frequency component and/or the high-frequency component. Such programmable filters allow a flexible and adaptive filtering of the desired frequencies, and thereby achieves a proper reconstruction even in cases where the frequency of either the low-frequency and/or the high-frequency is not static but may be variable. Thereby flexibility to drive different electrosurgical instruments having different requirements in terms of driving frequency can be met. Flexibility as well as ease of use of the electrosurgical generator is thus enhanced.

In an advantageous argument, a switchable blocking element for the low-frequency component is provided, preferably at the programmable filter of the low-frequency component. Thereby, extraction of low-frequency can be blocked. Thereby the electrosurgical generator is enabled to provide pulsed high-frequency energy as it is required for certain special driving modes, e.g. with pulses having a repetition time of about 3.7 us for a blend mode or of 5.5 µs for a spray/coagulation mode. As such repetition times correspond to frequencies within the ultrasound range, blocking of the low-frequency component ensures that all energy remains in the high-frequency path and will not be drained off in an unwanted manner to the low frequency portion. Thereby, compatibility of the electrosurgical generator according to the present invention with pulsed high-frequency modes is ensured.

Preferably, the frequency splitter comprises a demodulating device configured to extract the low-frequency component and a high-frequency component. By virtue of such a demodulation device the low-frequency component can be easily extracted from the carrier which is formed by the high-frequency component. This allows for an efficient re-generation of both frequency components.

Such a demodulating device is a preferred example of a reconstruction unit. Said reconstruction unit is advantageously located within the generator, preferably adjacent to the at least one output socket. This facilitates internal routing of the power conveying wiring from the inverter to the output socket and is beneficial for compact construction. However, although preferable, this is not a necessity. It is also possible to locate the reconstruction unit remotely which means outside of the electrosurgical generator, namely preferably at the electrosurgical instrument proper or at/along a cable which connects the electrosurgical instrument to the at least one output socket of the electrosurgical generator. Having the reconstruction unit located at or near the electrosurgical instrument has the benefit of a less complex connection cable to the instrument since owing to the modulation only a frequency range at or about the high-frequency needs to be conveyed as opposed to conventional cable connections. Those have to convey the low-frequency component as well as the high-frequency components, therefore have to serve a dual purpose which usually requires compromising. Having the reconstruction unit located at the instrument avoids such compromising and allows using of a cable specialized for conveying energy at (or around) the high-frequency only. Clearly, this avoids the necessity of routing two cables to the electrosurgical instrument as it is often encountered in the prior art. One cable that just needs to convey one specific frequency range (instead of two) completely suffices and allows usage of a simplified and therefore less complex cable that is easier to handle (e.g. features a lesser bending stiffness), thereby improving handling of the electrosurgical instrument.

Advantageously the low-frequency signal is a sinusoid signal, and preferably the high-frequency signal is another sinusoid signal having a frequency, and preferably phase, independent of the low-frequency signal. Using sinusoid signals in particular for the low-frequency signal allows for a reduction of harmonics which is beneficial in that less and easier filtering is required. Therefore, the energy of the low-frequency signal can be more effectively used. Further, according to the invention there is no specific relationship between frequency and phase of the high-frequency signals and the low-frequency signal, thereby avoiding any restriction on the frequency (as well as phase) of the high-frequency signal. This is a significant benefit over prior art systems that required a specific relationship between low and high-frequency signals, in particular demanded that the high-frequency signal shall be a harmonic of the low-frequency signal (e.g. EP 3 216 409 A1 requiring the high-frequency to be a harmonic of the low-frequency and further experiencing significant harmonics).

Preferably the modulation unit is configured for amplitude modulation. Such modulation can be readily achieved with rather simple and inexpensive components, and further allows for a simple demodulation. Moreover, such amplitude modulation has a benefit of requiring just a rather small bandwidth at or around the high-frequency carrier, in particular if the low-frequency signal is a sinus like signal. Having such a small bandwidth is beneficial for conveying the energy by a cable since the cable has to fulfill less stringent requirements only. However, the invention is not limited to amplitude modulation. It is also possible to employ modulation units that are configured for frequency or phase modulation (FM, PM) or other analog modulations, as well as digital modulations as they are known to a person skilled in the art. Contrary to common FM, preferably a carrier for the frequency (or phase) modulation is dynamically dependent on magnitude of the high-frequency signal. This allows for a more efficient amplification and/or a more precise reconstruction of the high-frequency signal.

Further, a modulation index in particular of the amplitude modulation is being limited to a presetable limit value. The modulation index defines the degree of varying the amplitude of the carrier based on a ratio of modulation excursions of the modulated signal to the level of the (unmodulated) carrier. It is expressed as a number, e.g. for a modulation index of m = 1.0 (also expressed as 100%) the wave amplitude of the modulated signal can shrink down to zero at times, and this represents full modulation. While this may be desirable for maximum ultrasound energy transfer, it is preferable to provide a maximum limit to the modulation index that is less than unity, preferably 0.7 or lower. By such a restricted limit it can be ensured that no overmodulation occurs and distortions induced by overmodulation can be prevented.

In a particular advantageous embodiment, a selectable bypass element is provided that is configured to bypass the modulation unit, preferably if an optional single frequency output mode is selected. In such a single frequency output mode just the low-frequency or and high-frequency is to be outputted but not both. In such a case there is no need for modulating one frequency on the other. Therefore, in particular if a low-frequency is to be employed it is beneficial to provide a bypassing element, thereby bypassing the modulation unit so that the low-frequency can (exclusively) be used directly without any interference by the (nonexisting) high-frequency. Therefore, in a pure low-frequency mode the electrosurgical generator is not hampered by in this case needless modulation and is thus capable of operating like a conventional ultrasound electrosurgical generator.

Advantageously, the low frequency driving unit and the high frequency driving unit are being formed as digital oscillators or, preferably as numerically controlled oscillators (NCO). Further preferably, the low-frequency driving unit, the high-frequency driving unit and the modulation unit as well as (if applicable) the bypass element are integrated into a field programmable gate array (FPGA). This allows for a compact and efficient generation of the required control signals.

In another particularly preferred embodiment that may deserve independent protection, a superpositioning circuit is provided in lieu of the modulation unit. This can for example be achieved by adding the low-frequency signal to the high-frequency signal, thereby achieving a combined reference signal that can be employed for controlling the inverter. While this is beneficial in that it requires less circuitry, there is still a signal component present in the baseband and therefore the benefit of a frequency shift to the high-frequency realized by using the modulation unit is not realized. So not all benefits will be realized by this variant, yet this is to be weighed against the advantage of simpler circuitry. The superpositioning circuit can e.g. be embodied as a summing circuit.

Preferably, the multilevel inverter comprises a plurality of inverter cells connected in a cascaded manner. Said cells are driven by the inverter controller. Such a cascaded configuration provides significant benefits in terms of generating various voltage levels. In particular, it is beneficial if some of the inverter cells operate at a higher DC level than others. This means there will be low voltage inverter cells (e.g. having a voltage of 12 V) and high voltage inverter cells (e.g. having a voltage of 48 V), thereby increasing voltage range of the multilevel inverter with a set number of inverter cells. However, other inverter cell arrangements may be employed, too, e.g. flying capacitor or clamped diode arrangements.

Typically, the multilevel inverter is configured in full bridge configuration. However, this not a must and the multi-level inverter may comprise cells configured in half-bridge configuration, thereby simplifying circuitry and reducing component count.

In a preferred embodiment, a spectral power of the output of the modulation unit in a frequency range above a frequency which is determined by a sum of the high frequency and the low frequency is lesser than a spectral power of a frequency band centered at the high frequency and having a width of two times the low frequency. Accordingly, the spectral power is focused at or around the high frequency, thereby avoiding the detrimental effect of having a lot of spectral power at higher harmonics. Having a lot of spectral power at higher harmonics is prone to wasting energy as well as increasing electrical disturbances due to higher electromagnetic emissions. Both unwanted effects can be reduced by concentrating the spectral power as claimed.

The invention is explained in more detail below by way of examples in conjunction with the accompanying drawing showing advantageous embodiments. In the drawing:
- Fig. 1: shows a frontal view of an electrosurgical generator with an attached hybrid electrosurgical instrument;
- Fig. 2: shows a block diagram of the electrosurgical generator;
- Fig. 3: shows a block diagram of a cascaded multi-level inverter;
- Fig. 4: shows a schematic functional diagram configuration of generating low and high frequency energy comprising a modulating device;
- Fig. 5A-D: show a low frequency signal, a high frequency signal, a modulated control signal for the multilevel inverter and a frequency spectrum;
- Fig. 6: shows a sample of a high frequency in a pulsated mode;
- Fig. 7: shows another embodiment wherein a reconstruction unit is located at the electrosurgical instrument or its cable; and
- Fig. 8A, B: show a variant to Fig. 4 having a superpositioning circuit in lieu of the modulating device.

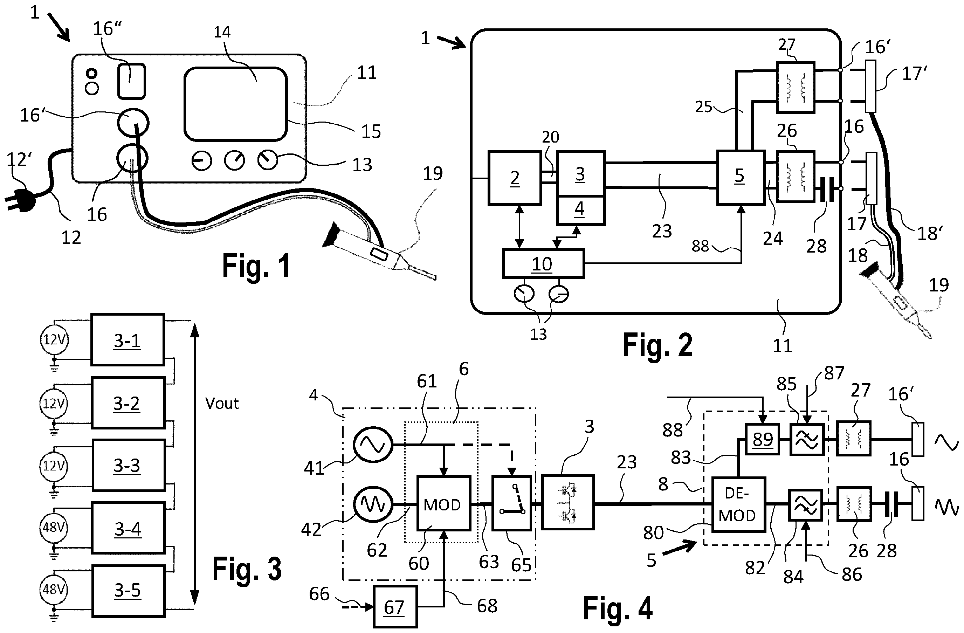

An electrosurgical generator according to an exemplary embodiment of the invention is illustrated in Fig. 1. The electrosurgical generator, identified as a whole by reference numeral 1, comprises a housing 11 having a plurality of output sockets 16, 16', 16" for connection of an electrosurgical instrument 19. In the depicted embodiment three output sockets are provided, comprising an HF output socket 16 for radiofrequency energy, an US output socket 16' for ultrasound energy, and a hybrid socket 16" which provides both, radiofrequency and ultrasound energy. A power supply cable 12 is provided with a plug 12' for connection to an electrical power source (not shown) which may be an electricity grid, like AC mains in a building, or an off-grid source of electric energy, like a 12 Volt or 24 Volt battery in a vehicle or mobile hospital. Further, a user interface 14 is provided comprising a display 15 which may be a touchscreen, or knobs 13 for inputs by a user.

As shown in Fig. 2, the electrosurgical generator 1 comprises in the housing 11 a power supply unit 2 that is supplied with electrical power by the mains connecting cable 12 (see Fig. 1). The power supply unit 2 comprises a rectifier and feeds a DC link 20 with direct voltage, the DC link 20 supplying an inverter 3. The inverter 3 generates alternating current in a selectable voltage range up to a few kilovolts at selectable frequencies up to 4000 kHz that is fed via an output line 23 to a reconstruction unit 5 having two outputs. At one output a high frequency output line 24 is connected leading via an isolation transformer 26 to the output socket 16 comprising a series capacitor 28 for blocking off any DC current, and at the other output of the reconstruction unit 5 a low frequency output line 25 is connected leading via a second isolation transformer 27 to the ultrasound output socket 16'. The electrosurgical instrument which is a hybrid electrosurgical instrument 19 to be supplied with radiofrequency energy as well as ultrasound energy 19 can be plugged into the output sockets 16, 16' by means of a HF cable 18 and an ultrasound cable 18', respectively.

The inverter generates high-frequency alternating voltage in the range between 20 kHz and 4 MHz. The inverter is embodied as a multi-level inverter 3. Shape, frequency, duty cyle and amplitude of the voltage generated by the multi-level inverter 3 are determined by an inverter control 4 based on reference signals which in turn are governed by the control unit 10 according to the power and mode as selected. The control unit 10 acts as a master controller which is connected to the user interface 14 such that the user can issue directions and commands for operation of the electrosurgical generator 1. The control unit 10 generates corresponding control signals and governs the relevant components, units and modules of the electrosurgical generator 1 according to these instructions and commands. In particular, the control unit 10 provides command signals to the inverter controller 4 which in turn controls the multi-level inverter 3 with its inverter cells.

The multi-level inverter 3 comprises a plurality of inverter cells 3-1 to 3-5 which are arranged in a cascaded formation as shown in Fig. 3. Each inverter cell 3-1 to 3-5 is supplied by a DC source of a set voltage (e.g. 12 Volt) at its input side (left side of each cell in Fig. 3) and generates an AC voltage that is output at its output side (right side of each cell in Fig. 3). In the depicted exemplary embodiment, the inverter cells 3-1 to 3-5 feature a half-bridge configuration. The respective outputs of the cells 3-1 to 3-5 are connected in series so that the voltages outputted by each of the cells 3-1 to 3-5 are added to a common output voltage Vout. In the depicted embodiment an optional grouping of the plurality of cells is shown. Accordingly, there are two different types of cells provided which are arranged in two groups which are supplied with different DC voltage. The first group comprises low-voltage cells 3-1, 3-2 and 3-3. They are supplied with a lower DC voltage, in the displayed embodiment 12 V. The second group comprises high-voltage cells 3-4, 3-5 which are supplied with a higher DC voltage, in the displayed embodiment 48 V. Both groups are connected in series to generate the common added output voltage Vout. Having such grouping of low and high voltage cells allows for achieving a higher number of voltage levels as opposed to having the same number of cells with identical voltage. Thereby, the configuration as shown can provide an output voltage ranging between -132 V and +132 V, which are equivalent to 23 levels in steps of 12 V. Accordingly, a finer "granularity" of the multilevel output of the multilevel inverter 3 can be achieved. The invention utilizes this for providing a modulated voltage as it will be explained in the following.

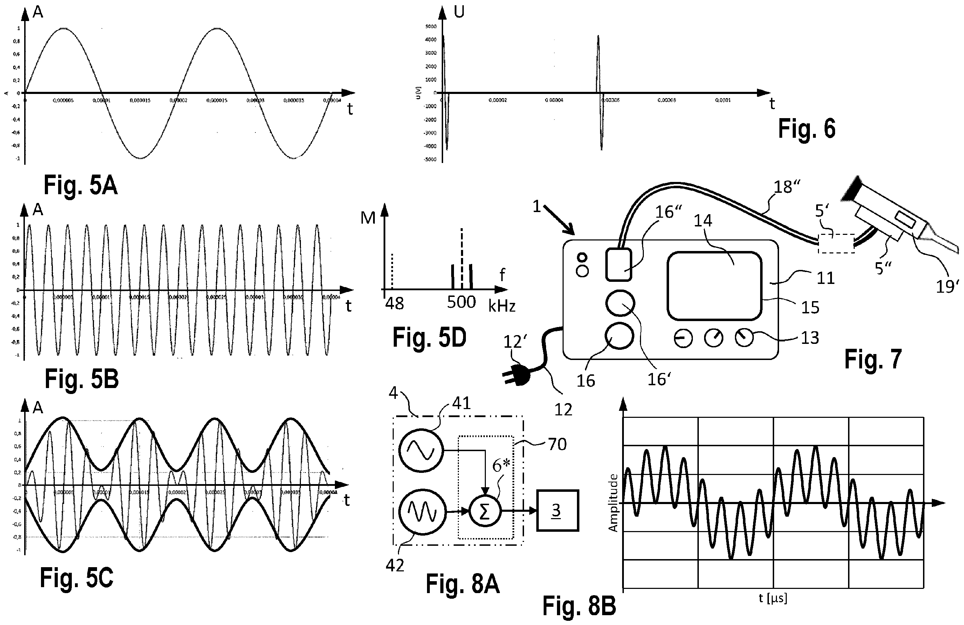

Shown in Fig. 4 is a block diagram of an exemplary embodiment of a modulation unit 6 of the inverter controller 4 and of the reconstruction unit 5 embodied as a frequency splitter 8. The inverter controller 4 comprises a low-frequency driving unit 41 that is configured for forming a low-frequency sinusoid signal, particularly in the ultrasound frequency range, to be used for driving an ultrasound electrosurgical instrument or an ultrasound portion of a hybrid electrosurgical instrument 19, and a high-frequency driving unit 42 that is configured for forming a high-frequency sinusoid signal, particularly in the radiofrequency range, to be used for driving an HF electrosurgical instrument or an HF portion of a hybrid electrosurgical instrument 19. Preferably, the sinusoid signals are pure sinus signals. As an example, the low-frequency driving unit 41 may generate a low-frequency signal of 48 kHz in the ultrasound range as depicted in Fig. 5A, and the high-frequency driving unit 42 may generate a signal of about 500 kHz as depicted in Fig. 5B which is in the radiofrequency range. It is to be noted that the high-frequency signal is not a harmonic of the low-frequency signal; it is an advantage of the invention that there is no restriction for the high-frequency to be a harmonic of the low-frequency.

The modulation unit 6 comprises an AM modulator 60 having an input 62 for a carrier to which the high-frequency signal of the high-frequency driving unit 42 is applied and an input 61 for a modulation signal to which the low frequency signal of the low-frequency driving unit 41 is supplied. The AM modulator 60 impresses the low-frequency modulation signal on the high-frequency carrier by varying the amplitude of the carrier in accordance with the modulation signal, thereby forming a modulated signal. The degree of varying the amplitude of the carrier is defined by a number m which is a modulation index. It is a measure based on a ratio of modulation excursions of the modulated signal to the level of the (unmodulated) carrier. For a modulation index of m = 1.0 (also expressed as 100%) the wave amplitude of the modulated signal can shrink down to zero at times, and this represents full modulation. While this may be desirable for high ultrasound energy transfer, it is preferable to provide a maximum limit to the modulation index that is less than unity, preferably 0.7 or lower. Such a presettable limit value may be applied from a limiter 67 via line 68 to the AM modulator 60. By such a limit, e.g. of 0.7, it is ensured that no overmodulation occurs and distortions induced by overmodulation can be prevented.

Accordingly, a modulated signal is formed as depicted in Fig. 5C and provided at an output 63 of the AM modulator 60. The modulated signal comprises three components as shown in the frequency domain of Fig. 5D: the carrier signal at 500 kHz being depicted by a broken line and two sidebands at 452 kHz and 548 kHZ being depicted as solid lines. For comparison, the modulation signal in the ultrasound frequency range of 48 kHz is shown as a dashed line, however it is no longer present in the modulated signal as a baseband signal rather it has been transformed to become the two sideband signals.

The inverter 3 receives the modulated signal as a reference signal for the desired waveform as shown in Fig. 5C. Owing to the inverter being a multilevel inverter it is capable of reproducing the desired waveform for generating a high-frequency alternating voltage. This is facilitated by employing the inverter cell arrangement as shown in Fig. 3 comprising two groups of cells each group having a different supply voltage, so that an inverter cell 3-1 to 3-3 of the first group supplied with a lower DC voltage is to be used if a lower voltage change is desired or that an inverter cell 3-4 or 3-5 of the second group supplied with a higher DC voltage 34 is to be used if a larger change of AC voltage amplitude is desired. For details of such usage of various groups of inverter cells being supplied with different DC voltage in a multilevel inverter reference is made to pending patent application DE 10 2021 122 284 A1 of the present applicant which is herein incorporated by reference. Accordingly, the multilevel inverter 3 generates instantly and in one go, without any mixing on the high-voltage energy level, the required high-frequency alternating voltage conveying ultrasound and HF energy to be supplied by the outputs 16, 16', 16" of the electrosurgical generator 1 for driving the electrosurgical instrument 19.

As it can be readily appreciated, the output line 23 has to convey high-frequency energy only and thus needs to be specifically adapted to the frequency in the radiofrequency range of about 500 kHz only; it is of no importance how that connection performs in the low-frequency range. This facilitates optimization of the output line 23 and cable 18, 18', 18" to the instrument in particular with respect to efficient power conveying without unwanted reflections (e.g. improved standing wave ratio) .

Further, as it can be readily appreciated in Fig. 5D, a spectral power of the output of the inverter 3 is zero above the frequency of the upper sideband (said frequency being determined by the sum of the carrier frequency and the modulation signal frequency) and is thus much less than a spectral power comprised at the carrier up to and including its lower and upper sideband. Accordingly, the are no unwanted harmonics beyond the upper sideband which improves purity of the sinus-like voltage of the modulated high-frequency energy used to drive the electrosurgical instrument.

The output line 23 conveys the modulated high-frequency energy to the output sockets 16, 16' via a reconstruction unit 5 that is arranged within the electrosurgical generator in the embodiment as depicted in Fig. 2. The reconstruction unit 5 and further comprises, as shown in Fig. 4, a frequency splitter 8, a first programmable filter 84 for high-frequency in the radiofrequency range and a second programmable filter 85 for the ultrasound frequency range. Subsequent to the reconstruction unit 5 isolation transformers 26, 27 are provided for the output socket 16, 16', respectively. The isolation transformers 26, 27 serve the purpose of a galvanic isolation and further could be configured such as to provide a step up of output voltage, thereby becoming stepping-up transformers, too. Further, at least prior to the output socket 16 for the energy in the radiofrequency range a safety capacitor 28 is provided that is configured to block any DC current for protection of the patient.

The frequency splitter 8 comprises a demodulating device 80 which is configured to demodulate the modulated high-frequency energy supplied by the output line 23 to an input of the demodulating device 80. The demodulating device 8 may be embodied as a simple envelope detector or as a more sophisticated synchronous detector, for example a product detector, which has advantages in terms of reliable and robust demodulation even in case of a high modulation index, in particular a modulation index reaching unity (or even higher). Subsequent to demodulation, the demodulating device 80 outputs the low-frequency energy at output 83 and the high-frequency energy from the carrier at output 82. Said output 82 leads to a programmable filter 84 which may be configured as a high pass or as a bandpass at or around the carrier frequency. Thereby unwanted frequency components are cut off and the energy of the carrier is conveyed via the isolation transformer 26 and the safety capacitor 28 to the HF output socket 16. Likewise, the demodulated low-frequency energy is provided at output 83 and conveyed via a programmable filter 85 which may be configured as a low-pass or a bandpass around the ultrasonic frequency and via the second isolation transformer 27 to the ultrasound output socket 16'. Connected to the output sockets 16, 16' are plugs 17, 17' of cables 18, 18' for HF energy and ultrasound energy which are leading the respective HF energy and ultrasound energy to the (hybrid) electrosurgical instrument 19. Thereby, the electrosurgical instrument 19 is provided with the energy in the radiofrequency range as well as in the ultrasound frequency range as required for proper operation.

Further, at the programmable filter 84 an input 86 for programming a roll-off or center frequency is provided. Thereby, adjustments to the filter characteristics can be achieved, thus allowing an adaption of the programmable filter 84 to different electrosurgical instruments 19, 19' or different modes of operation of the electrosurgical generator 1 and/or the electrosurgical instrument 19, 19'. Likewise, the programmable filter 85 is provided with an input 87 for programming a roll-off or center frequency. Further, at the programmable filter 86 a switchable optional blocking element 89 is provided. It is switched on or off by an activation line 88 by the control unit 10. This switching on (or off) is performed by the control unit 10 dependent on the mode of operation as selected by the user interface 14, e.g. by means of the knobs 13. This is of particular importance for modes like the one shown in Fig. 6 which provides HF energy in brief pulses which are repeated at a rate of about 3.7 µs for a blend mode or of 5.5 µs for a spray/coagulation mode. Such a rate is well within the ultrasound frequency. In order to block any unwanted energy leakage into the ultrasound branch to the ultrasound socket 16', the switchable blocking element 89 is activated by the control unit 10 via activation line 88. Conversely, if the selected mode calls for delivery of ultrasound energy, the control unit 10 deactivates the blocking element 89 so that ultrasound energy can flow from the demodulating device 80 to the ultrasound output socket 16'.

In the embodiment shown in Fig. 2 the reconstruction unit 5 is located within the housing 11 of the electrosurgical generator 1, typically adjacent to the output sockets 16, 16'. Alternative embodiments having different locations of the reconstruction unit are shown in Fig. 7. Accordingly, the reconstruction unit 5' may be located at the cable 18" supplying the electrosurgical instrument, preferably near the electrosurgical instrument. Alternatively, the reconstruction unit 5" may be located at or in the electrosurgical instrument 19' itself, thereby allowing the modulated energy to be conveyed directly to or at least close to the electrosurgical instrument 19'.

Optionally, a switchable bypass element 65 is provided that is configured to bypass the modulation unit 60, preferably if an optional single frequency output mode for ultrasound energy only is selected. As only the low-frequency is to be outputted there is no need for modulating said low-frequency on a high frequency. Therefore in such a mode, said bypass element 65 is activated by the control unit 10 for bypassing the AM modulator 60 so that the low-frequency can be used directly for controlling the inverter without any interference by the high-frequency. Therefore, needless modulation is avoided and the electrosurgical generator is operated like a conventional ultrasound electrosurgical generator.

A variant to the embodiment of Fig. 4 is depicted in Fig. 8A. Accordingly, a superpositioning circuit 70 is provided replacing the AM modulator 60. It combines the reference signals of the low-frequency driving unit 41 and of the high-frequency driving unit 42 by means of a summing circuit 6*. The resulting combined reference signal is shown in Fig. 8B. It is supplied to the multilevel inverter 3 which accordingly amplifies this to generate energy for driving the electrosurgical instrument 19 having spectral components as shown by dashed and dotted lines in Fig. 5D. The reconstruction unit 5 is facilitated, too as no demodulating device 80 is required and the energy provided by the inverter 3 can be supplied directly to the programmable filters 84, 85. So the reconstruction unit is basically identical to that shown in Fig. 4, however with removed demodulating device 80.

## Claims

1. Electrosurgical generator configured to provide a dual frequency power output in a low frequency range as well as in an high frequency range to at least one electrosurgical instrument (19), comprising a control unit (10) and an inverter unit configured to simultaneously generating said dual frequency power output that is supplied to at least one output socket (17, 17') for connection of at least one electrosurgical instrument (19, 19'),
**characterized in that**
the inverter unit is configured as a multilevel inverter (3) being driven by an inverter controller (4), said inverter controller comprising:
- a low frequency driving unit (41) configured for forming a low frequency reference signal for the low frequency output of the electrosurgical generator,
- a high frequency driving unit (42) configured for forming a HF reference signal for the high frequency output of the electrosurgical generator, and
- a modulation unit (6) configured for forming a modulated combined reference signal by modulating the low frequency reference signal onto the high frequency reference signal, wherein the modulated combined reference signal is supplied to the multilevel inverter (3) for generating the dual frequency power output.

2. Electrosurgical generator according to claim 1, wherein the low frequency is in the ultrasound frequency range and the high frequency is in the radiofrequency range.

3. Electrosurgical generator according to claim 1 or 2, wherein a reconstruction unit (5) is provided comprising a frequency splitter (8) having two outputs, a first output (24) connected to a high frequency supply line and a second output (25) connected to a low frequency supply line, the supply lines being configured for supplying the output power to the at least one electrosurgical instrument (19).

4. Electrosurgical generator according to the preceding claim, wherein the frequency splitter (8) comprises programmable filters (84, 85) for the high frequency component and/or the low frequency component.

5. Electrosurgical generator according to the preceding claim, wherein a switchable blocking element (89) for the low frequency component is provided, preferably at the programmable filter (85) of the low frequency component.

6. Electrosurgical generator according to any of claims 3 to 5, wherein the frequency splitter (8) comprises a demodulating device (80) configured to extract a low frequency component and a high frequency component of the power output.

7. Electrosurgical generator according to any of claims 3 to 6, wherein the reconstruction unit (5) is located within the generator, preferably adjacent to the at least one output socket.

8. Electrosurgical generator according to any of claims 3 to 6, wherein the reconstruction unit (5', 5") is located remotely at the electrosurgical instrument (19') or at a cable (18") connecting the electrosurgical instrument (19') to the at least one output socket of the electrosurgical generator.

9. Electrosurgical generator according to any of the preceding claims, wherein the low frequency signal is a sinusoid signal, and preferably the high frequency signal is another sinusoid signal having a frequency, and preferably phase, independent of the low frequency signal.

10. Electrosurgical generator according to any of the preceding claims, wherein the modulation unit (6) is configured for amplitude modulation, preferably with a modulation index being limited to a presetable limit value of less than unity, further preferably at a maximum of 0.7 or lower.

11. Electrosurgical generator according to any of the preceding claims, wherein a selectable bypass element (65) is provided configured to bypass the modulation unit (6), preferably if an optional single frequency output mode is selected.

12. Electrosurgical generator according to any of the preceding claims, wherein a superpositioning circuit (70) is provided in lieu of the modulation unit.

13. Electrosurgical generator according to any of the preceding claims, wherein the multilevel inverter (3) comprises a plurality of inverter cells (3-1, 3-2, 3-3, 3-4, 3-5) connected in a cascaded manner that are driven by the inverter controller (4).

14. Electrosurgical generator according to any of the preceding claims, wherein the multilevel inverter (3) is configured comprising cells in half-bridge configuration.

15. Electrosurgical generator according to any of the preceding claims, wherein a spectral power of the output of the modulation unit (6) in a frequency range above a frequency, which is determined by a sum of the high frequency and the low frequency, is lesser than a spectral power of a frequency band centered at the high frequency and having a width of two times the low frequency.
